# EUROPEAN PATENT APPLICATION

(11) **EP 3 900 846 A1**
(43) Date of publication of application: **27.10.2021**
(21) Application number: 20170625.6
(22) Date of filing: 21.04.2020
(51) Int. Cl.: B06B 1/02, A61B 8/12, B06B 1/06, G01S 7/52

(54) **ACOUSTIC IMAGING PROBE WITH A TRANSDUCER ELEMENT**

(71) Applicant: Koninklijke Philips N.V., 5656 AG Eindhoven (NL)
(72) Inventor: JACOBS, Egbertus Reinier, 5656 AE Eindhoven (NL)
(74) Representative: Philips Intellectual Property & Standards

(57) **Abstract**

An acoustic imaging probe having an adjustable effective elevation length. The acoustic imaging probe has a transducer element, comprising a plurality of acoustic transducers, that is divided into a plurality of sets of adjacent transducers. A processing module controls how many sets contribute to an acoustic pulse emitted by the acoustic transducer element during an imaging process, to thereby adjust an effective elevation length of the acoustic imaging probe.

## Description

### FIELD OF THE INVENTION

The present invention relates to the field of acoustic imaging probes, and in particular to acoustic imaging probes having a transducer element.

### BACKGROUND OF THE INVENTION

Acoustic imaging probes comprising an array of transducer elements are being increasingly used in the medical field. One use case scenario for such an imaging probe is with an intravascular catheter (e.g. an IVUS), in which transducer elements are positioned on (distal end of) a catheter for insertion into a blood vessel of a subject. Typically, a transducer element is formed of one or more separate transducers or "drums". Each transducer may comprise, for example, a piezoelectric transducer (PZT) or a capacitive micromachined ultrasonic transducer (CMUT).

Typically, a transducer element emits an acoustic pulse, such as an ultrasound pulse, and detects echoes of the emitted acoustic pulse. The echoes can be processed to generate an image. The acoustic pulse may be an ultrasound pulse, for which the corresponding generated image is an ultrasound image.

There is an ongoing desire to maximize a resolution of images generated by an array of transducer elements. In some existing solutions, this is achieved by operating the transducers of each transducer element at a higher center frequency. However, this comes at the cost of reduced penetration depth of the acoustic pulse(s). Some solutions propose to circumvent this problem by making the array operable in a high resolution mode (which has a reduced penetration) and a high penetration mode (which has a reduced resolution). In these solutions, the two modes are achieved by shifting the center frequency of the acoustic pulse emitted by the transducers of the array from a high frequency, for the high resolution mode, to a low frequency, for the high penetration mode.

There is an ongoing desire to provide alternative and/or improved mechanisms for achieving high penetration and/or high resolution.

### SUMMARY OF THE INVENTION

The invention is defined by the claims.

According to examples in accordance with an aspect of the invention, there is provided an acoustic imaging probe.

The acoustic imaging probe comprises: a transducer element, the transducer element comprising: a first set of one or more adjacent transducers; and a second set of one or more adjacent transducers, adjacent to the first set of one or more transducers, wherein each transducer is configured to controllably emit an acoustic pulse and receive one or more echoes responsive to the emitted acoustic pulse.

The acoustic probe further comprises a processing module configured to control the operation of the transducer element, the processing module being configured to be operable in at least: a first mode, in which the processing module controls the transducer element so that only the first set of one or more adj acent transducers emits an acoustic pulse and the transducers of the first set operate synchronously when emitting an acoustic pulse to provide a first combined acoustic pulse; and a second mode, in which the processing module controls the transducer element so that both the first and second sets of one or more adjacent transducers emit acoustic pulses and the transducers of the first and second sets operate synchronously when emitting an acoustic pulse to provide a second combined acoustic pulse.

The present invention provides an acoustic probe having one or more transducer elements, formed of adjacent transducers, where each transducer element has a controllable effective length. The transducers of the transducer element may be positioned along a same axis, i.e. form a linear array. In particular, by dividing the transducer element into two or more adjacent sets (each set being individually controllable by a processing module), the number of active sets controls the effective length or size of the transducer element.

The inventor has recognized that penetration depth of an acoustic pulse can be increased by operating more transducers simultaneously, so that a combined acoustic pulse, emitted by an transducer element having a greater effective length, has a greater magnitude or more acoustic pressure (than a shorter transducer element) leading to greater depth penetration. However, the inventor has recognized that an increased length/size of the transducer element, to provide such additional transducers, reduces a possible resolution of an acoustic image generated from echoes of the acoustic pulse. This is because, with a longer or larger transducer element, it is more difficult to pinpoint a precise location of an element that rebounds an acoustic pulse, as the precise origin of the acoustic pulse becomes less certain as the transducer element increasingly diverges from a point source to a linear/area source (i.e. gets longer or larger).

The inventor proposes to operate a transducer element in two modes. In a first mode, a first (relatively small) portion of the full transducer element is controlled to emit the combined acoustic pulse. In a second mode, a second, larger portion of the full transducer element is controlled to emit the combined acoustic pulse.

In this way, when operating in the first mode, imaging resolution can be improved (as the effective length/size of the transducer element is reduced) at the expense of reduced penetration (due to the use of fewer transducers). When operating in the second mode, penetration depth can be increased (due to the use of more transducers), at the expense of reduced imaging resolution (as the length/size of the transducer element increases). The proposed acoustic probe thereby allows thereby benefits from a more flexible operation, thereby increasing its utility and avoiding the need to provide separate probes for different penetration/resolution requirements (at additional cost/material requirements and potentially requiring multiple invasive procedures to appropriately image a subject).

It would be particularly advantageous to use a linear transducer element, as the effect of changing the elevation length is particularly noticeable in a linear transducer element. A concept of switching between the first and second modes is of particular use for linear arrays, as they are commonly used in scenarios in which both high and low penetration information would be desirable (e.g. in intravascular ultrasound devices).

The acoustic imaging probe may, of course, comprise a plurality of transducer elements, each configured in the manner of the previously described transducer element.

In some embodiments, each transducer is configured to require a voltage bias to controllably emit an acoustic pulse, and the processing module is configured to: when operating in the first mode, provide a voltage bias to only the first set of adjacent transducers; and wherein operating the second mode, provide a voltage bias to the first and second sets of adjacent transducers.

In some examples, the processing module is configured to control the transducer element so that a center frequency of the first combined acoustic pulse is different to the center frequency of the second combined acoustic pulse.

In at least one embodiment, the processing module is configured to control the transducer element so that a center frequency of the first combined acoustic pulse is greater than the center frequency of the second combined acoustic pulse.

The inventor has recognized that a larger difference between the first and second modes can be provided by switching the frequency at the same time as switching the number of transducers that emit the combined acoustic pulse. Thus, there is a combined effect of switching the frequency and the number of (adjacent) transducers used.

The processing module may be further configured to be operable according to a combination strategy, in which the processing module iteratively switches between operating in the first mode and operating in the second mode.

When operating according to the combination strategy, the processing module may switch between the first and second mode in response to a predetermined number of combined acoustic pulses being emitted by the transducers.

For example, the processing module may switch from the first mode to the second mode (and vice versa) in response to a single combined acoustic pulse being emitted by the transducer (since a last switch), i.e. after each combined acoustic pulse. In other examples, the processing module may switch from the first mode to the second mode (and vice versa) in response to a predetermined number of combined acoustic pulses, e.g. 2, 3 or 5, being emitted by the transducers (since a last switch).

Of course, the predetermined number of combined acoustic pulses may be different when switching from the first mode to the second mode as from when switching from the second mode to the first mode. For example, the processing module may switch from the first mode to the second mode in response to a first predetermined number of first combined acoustic pulses being emitted by the transducers, and may switch from the second mode to the first mode in response to a second predetermined number of second combined acoustic pulses being emitted by the transducers. The first and second predetermined numbers may be different. This embodiment increases the flexibility of imaging, and allows greater control over the power consumption of the imaging probe (as use of more transducers increases a power consumption of the imaging array).

Optionally, the transducer element further comprises a third set of one or more adjacent transducer, adjacent to the second set of one or more transducers; the processing module is configured so that, when operating in the second mode, only the first and second sets of one or more adjacent transducers emit acoustic pulses and the adjacent transducers of the first and second sets operate synchronously when emitting an acoustic pulse to provide a third combined acoustic pulse; and the processing module is further operable in a third mode, in which the processing module controls the transducer element so that the first, second and third sets of one or more transducers emit acoustic pulses and the transducers of the first, second and third sets operate synchronously when emitting an acoustic pulse.

This embodiment increases the flexibility of the imaging probe to have more granular control over the penetration depth and/or imaging resolution, to thereby facilitate selection of an appropriate compromise for a desired imaging operation.

Of course, the transducer element may comprise more than three sets of adjacent transducers, and the processing module may be operable in further modes to control the effective length of the transducer element through appropriate control over the number of sets of adjacent transducers that are capable of contributing to the combined acoustic pulse.

Preferably, when operating in any mode, each set of transducers that contributes to the emitted combined acoustic pulse is adjacent to another one of the sets of transducers that contributes to the emitted combined acoustic pulse.

Where more than two modes are available to the processing module, the processing module may operate according to different combination strategies, which define how the processing module switches between the different modes. Various patterns for such combination strategies would be considered by the skilled person, but typically comprise moving between at least three different modes according to a predefined switching scheme.

In some embodiments, the processing module is adapted to monitor the first and second sets of transducers to generate a receive signal responsive to the one or more echoes received by the first and second sets of transducers.

In some examples, each transducer may comprise a capacitive micromachined ultrasonic transducer, CMUT. In other examples, each transducer is a piezoelectric transducer, PZT. Other examples of suitable transducers will be known to the skilled person.

Optionally, the mode in which the processing module operates (or, where relevant, the combination strategy that the processing module operates according to) is responsive to a user input signal. Thus, a user may be able to control in which mode or optionally combination strategy the processing module operates.

The acoustic imaging probe may comprise a plurality of transducer elements, each comprising a first and second set of transducers, wherein the processing module is configured to control the operation of each of the plurality of transducer elements, wherein: when operating in the first mode, the processing module controls each transducer element so that only the first set of one or more adjacent transducers of each transducer element emits an acoustic pulse and the transducers of the first set of each transducer element operate synchronously when emitting an acoustic pulse to provide a first combined acoustic pulse; and when operating in the second mode, the processing module controls each transducer element so that both the first and second sets of one or more adjacent transducers of each transducer element emit acoustic pulses and the transducers of the first and second sets of each transducer element operate synchronously when emitting an acoustic pulse to provide a second combined acoustic pulse.

According to examples in accordance with an aspect of the invention, there is computer-implemented method of controlling an acoustic imaging probe comprising a transducer element, the transducer element comprising: a first set of one or more adjacent transducers; and a second set of one or more adjacent transducers, adjacent to the first set of one or more transducers, wherein each transducer is configured to controllably emit an acoustic pulse and receive one or more echoes responsive to the emitted acoustic pulse.

The computer-implemented method comprises: controlling the transducer element using at least two modes, including: a first mode, in which the processing module controls the transducer element so that only the first set of one or more adjacent transducers emits an acoustic pulse and the transducers of the first set operate synchronously when emitting an acoustic pulse to provide a first combined acoustic pulse; and a second mode, in which the processing module controls the transducer element so that both the first and second sets of one or more adjacent transducers emit acoustic pulses and the transducers of the first and second sets operate synchronously when emitting an acoustic pulse to provide a second combined acoustic pulse.

The skilled person will appreciate that the computer-implemented method may be appropriately adapted to carry out any embodiment of the invention described with reference to the embodiments of the disclosed acoustic imaging probe.

There is also proposed a computer program product comprising computer program code means which, when executed on a computing device having a processing system, cause the processing system to perform all of the steps of any herein described method.

There is also proposed a processing module configured to perform any herein described method.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates an acoustic imaging device;
Figure 2 is a block diagram illustrating an acoustic imaging probe according to an embodiment of the invention;
Figure 3 illustrates an acoustic imaging probe;
Figure 4 illustrates a method according to an embodiment;
Figure 5 illustrates a method according to an embodiment; and
Figure 6 illustrates an ultrasound imaging system in which an embodiment of the invention can be implemented.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an acoustic imaging probe having an adjustable effective elevation length. This is achieved by having dividing an acoustic transducer element into a plurality of sets of adjacent transducers, and controlling how many sets contribute to an acoustic pulse emitted by the acoustic transducer element during an imaging process.

The present invention relies on the recognition that a change in elevation length can be achieved by controlling how many sets of transducers contribute to a combined acoustic pulse emitted by the transducer element. This results in a tunable acoustic imaging probe, which can switch between a high-resolution (low penetration) mode and a high-penetration (low resolution) mode.

Embodiments of the invention can be employed in any acoustic imaging system, such as an ultrasound imaging system. Particularly advantageous embodiments are employed in an intravascular ultrasound imaging system.

Typically, acoustic pulses are ultrasound pulses, although other forms of acoustic pulses are possible and envisaged. Thus, the term "acoustic" in this disclosure may be replaced by the term "ultrasound" where relevant to achieve an embodiment of the invention (e.g. an ultrasound imaging probe is an example of an acoustic imaging probe).

For the avoidance of all doubt, the "transducer element" of the present invention is a (linear) array of one or more separate, but adjacent, transducers (such as a PZT or CMUT) that operate and are controlled synchronously. An imaging probe usually comprises an array of such transducer elements, which are appropriately controlled to image a desired volume. Alternative labels for a transducer element may be a transducer set, a transducer group or an array of transducers.

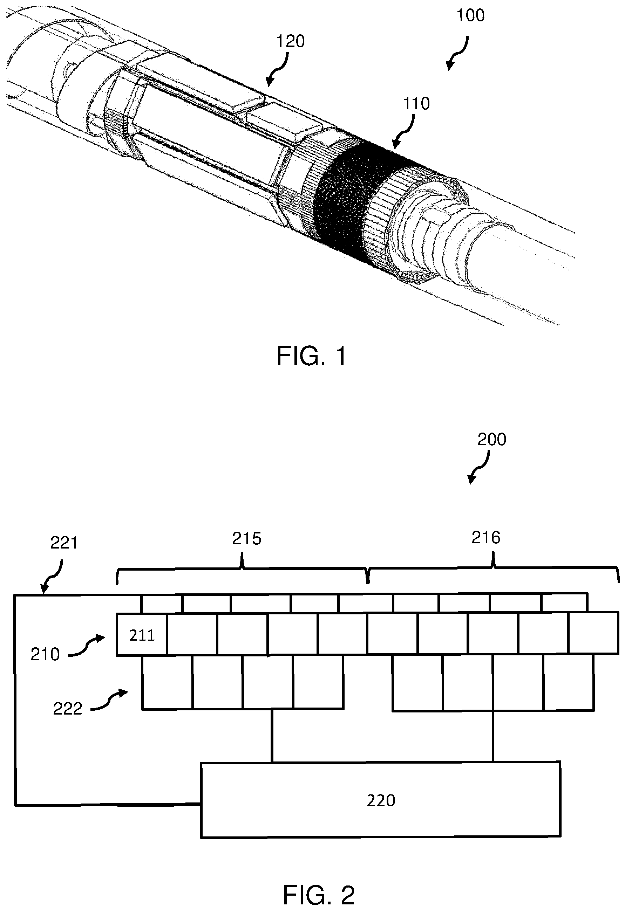

Figure 1 illustrates an example of an acoustic imaging probe 100, which here is an intravascular acoustic imaging probe for an intravascular ultrasound (IVUS) system. Although the exemplary description is related to intravascular acoustic imaging probe, the invention is applicable for other interventional medical devices, such as interventional needle.

The acoustic imaging probe 100 comprises a transducer element 110, which may be a linear array of transducers or "drums", which are controlled by a processing module 120. The processing module 120 may comprise, for example, an integrated circuit such as an ASIC or FPGA, as would be known by the skilled person.

The operation of an acoustic imaging probe 100 will be well known to the skilled person. Briefly, the acoustic imaging probe 100 operates by the processing module 120 controlling the individual transducers of the transducer element 110 to emit an acoustic pulse, any echoes or reflections of which are then detected by the transducer element. In particular, each transducer of a transducer element is synchronously controlled to emit an individual acoustic pulse, which, when the transducers are operated synchronously, results in the transducer element emitting a singular combined acoustic pulse.

Control of the transducer element 110 may be performed in a linear manner (e.g. in which all transducer elements emit an acoustic pulse simultaneously) or in a phased driven manner (e.g. in which transducer elements emit acoustic pulses at different times). Methods of appropriately controlling an array of transducers forming the transducer element will be apparent to the skilled person.

The processing module 120 then processes a signal generated by the transducer elements (responsive to the received echoes/reflections) to identify the presence and/or shape/appearance of the object(s) that reflect(s) the emitted acoustic pulse. This information may undergo further processing to generate an acoustic image, such as an ultrasound image.

A more complete description of a possible process and system for generating and processing acoustic pulses, e.g. to generate an acoustic image, will be provided later in this disclosure.

Figure 2 diagrammatically illustrates, in a block-diagram format, elements of an acoustic imaging probe 200 according to an embodiment of the invention.

The acoustic imaging probe 200 comprises a transducer element 210 formed of a plurality of transducers 211 arranged in an array. The transducer element 210 is here formed as a linear array, but other embodiments are possible (e.g. a 2-dimensional array). The transducer element 210 is divided into two sets 215, 216 of one or more adjacent transducers, which can be identified as a first set 215 and a second set 216 of transducers. The two sets are adjacent to one another.

The transducer element 210 may be divided into more than two (adjacent) sets of adjacent transducers, although only two sets are presently illustrated for the ease of understanding.

The acoustic imaging probe 200 also comprises a processing module 220. The processing module 220 is adapted to perform the process previously described. In particular, the processing module 220 is adapted to control, e.g. using a control line 221, the operation of the transducer element 210. The processing module 220 thereby controls the transducer element 210 to emit an acoustic pulse.

As previously mentioned, in typical imaging probes, each individual transducer of an array of transducer elements is synchronously controlled to emit an acoustic pulse, e.g. using the control line 221. The same control line is provided to each individual transducer, so that they are controlled synchronously. The acoustic pulse output by the array is a combination of all acoustic pulses that are synchronously emitted by the individual transducers which form a single "combined acoustic pulse".

The proposed acoustic imaging probe is slightly modified to enable different numbers of transducers 211 to contribute to the combined acoustic pulse emitted by the transducer element 210.

The processing module 220 is configured to be operable in at least two modes.

In a first mode, the processing module 220 controls the transducer element 210 so that only the first set 215 of transducers contributes to the acoustic pulse emitted by the transducer element 210. In other words, the processing controls the transducer element so that only the first set of one or more adj acent transducers emits an acoustic pulse and the transducers of the first set operate synchronously when emitting an acoustic pulse to provide a first combined acoustic pulse. Thus, the second set 216 do not contribute to the combined acoustic pulse in the first mode.

In a second mode, the processing mode controls the transducer element 210 so that both the first set 215 and the second set 216 of transducers contribute to the acoustic pulse emitted by the transducer element. In other words, the processing module controls the transducer element so that both the first and second sets of one or more adjacent transducers emit acoustic pulses and the transducers of the first and second sets operate synchronously when emitting an acoustic pulse to provide a second combined acoustic pulse.

In this way, the effective size or length of the transducer element 210 (when emitting an acoustic pulse) can be modified by the processing module 220. This enables the processing module to control the effective elevation length of the transducer element 210.

In the illustrated example, each transducer is a capacitive micromachined ultrasonic transducer (CMUT). A CMUT requires a voltage bias, e.g. provided by a voltage bias line 222, to operate effectively (e.g. to emit an acoustic pulse that can contribute to the overall acoustic pulse emitted by the transducer element 210). Unbiased transducers will also have a negligible response to reflections/echoes of an emitted acoustic pulse. In particular, CMUTs become insensitive to received echoes (or any other acoustic transmission) when they are not in collapse after transmitting an acoustic pulse.

In the illustrated example, the processing module 210 controls which sets of transducers are able to contribute to the acoustic pulse emitted by the transducer element 210 by controlling which transducers are provided with a voltage bias. This is achieved by providing a separate voltage bias line for each set 215, 216 of transducers, and controlling which voltage bias lines carry a voltage bias depending upon the mode of the processing module.

Thus, the processing module 220 may be configured to: when operating in the first mode, provide a voltage bias to only the first set of adjacent transducers; and wherein operating the second mode, provide the voltage bias to the first and second sets of adjacent transducers.

Other methods for controlling which transducers are active, e.g. are able to contribute to the acoustic pulse emitted by the transducer element 210 will be apparent to the skilled person. For example, the processing module may provide separate control lines 221 for each set of acoustic elements. This approach enables other types of transducers, such as those that do not rely upon a voltage bias such as a piezoelectric transducer, to be used as the transducers of the transducer element 210.

Where possible, it is preferable to control which transducers are able to contribute to the acoustic pulse emitted by the transducer element using voltage bias lines. This is because controlling using separate control lines may introduce significantly more interconnects than voltage bias lines (e.g. due to the need for precise control and monitoring of the control lines - compared to a voltage bias line where this precision is less essential).

Controlling using voltage bias lines would be particularly advantageous if the invention were implemented in an IVUS acoustic probe, to minimize the size of the IVUS acoustic probe (which is an important consideration for such probes, to minimize patient discomfort and potential injury).

The processing module 220 may be configured to control a center frequency of the emitted acoustic pulse. Methods of controlling a center frequency of an acoustic emitted by an individual transducer will be apparent the skilled person, e.g. through appropriate control of a signal transmitted over the control line 221.

Preferably, the processing module 210 is configured to control the transducer element so that a center frequency of acoustic pulse ("first combined acoustic pulse") emitted by the transducer element when operating in the first mode is different to the center frequency of the acoustic pulse ("second combined acoustic pulse") emitted by the transducer element when operating in the second mode.

In particular, the processing module is configured to control the transducer element so that a center frequency of the first combined acoustic pulse is greater than the center frequency of the second combined acoustic pulse. This increase a penetrative depth of the acoustic pulse when operating in the second mode, and increases a resolution of the imaging prove when the processing module operates in the first mode, thereby increasing an effect of switching between the first and second modes.

Where the transducer element 210 is divided into more than two sets of transducers, the processing module may be adapted to be operable in more than two modes, e.g. to increase the range of effective elevation lengths for the transducer element 210. For example, the second mode may comprise controlling the transducer element so that only a first and second set contribute to the acoustic pulse emitted by the transducer element, and there may a third mode in which the processing module controls the transducer element so that the first, the second and a third set of acoustic elements contribute to the acoustic pulse emitted by the transducer element 210.

Preferably, in any operating mode of the processing module, all sets of acoustic elements that contribute to the acoustic pulse emitted by the transducer element are adjacent to at least one other of the sets of acoustic elements that contribute to the acoustic pulse emitted by the transducer element. This ensures that the elevation length of the transducer element can be controlled with minimal reduction of resolution.

Although Figure 2 illustrates only 10 transducers, e.g. 5 transducers in each of the first 215 and second 216 sets of transducers, embodiments may comprise any number of transducers. The total number of transducers may be in the region of 10 to 100, e.g. between 5 and 50 transducers per set (assuming there are only two sets).

Preferably, each set of transducers comprises at least 2 transducers, for example, at least 10 transducers, e.g. between 10 and 15 transducers. This provides a good balance between penetration and resolution for the different modes.

Of course, the number of transducers in each set does not need to be identical. In some embodiments, the number of transducers in the second set is greater than the number of transducers in the first set, to increase the effect of switchability between high resolution (and low penetration) and high penetration (but low resolution) operating modes.

The precise number of transducers in each set of transducers may differ depending upon implementation details, namely dependent upon the desired penetration and/or resolution of imaging in each mode.

Although in Figure 2 the first set 215 and the second set 216 are illustrated laterally adjacent to each other, various alternative exemplary embodiments may comprise a first and second set adjacent to each other in longitudinal direction of a medical interventional device on which they are mounted (e.g. intravascular imaging probe). In a further alternative embodiment, respective transducers of the first set and the second set are in each other's prolongation along the longitudinal dimension of the transducers.

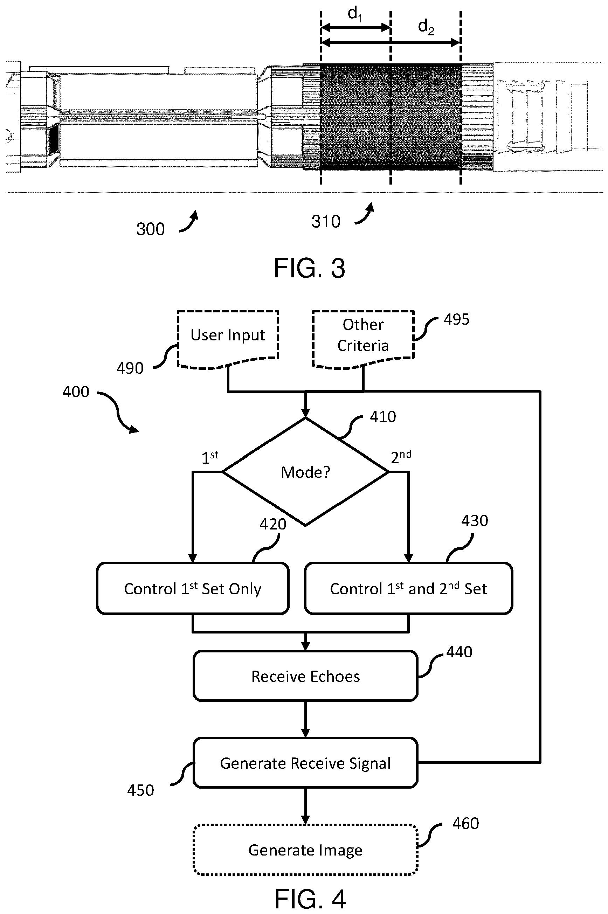

Figure 3 illustrates the effect of controlling different numbers of the sets of the transducers of the transducer element 310 of the acoustic imaging probe 300.

When the processing module operates in the first mode, and only a first set of the transducers contributes to the acoustic pulse emitted by the transducer element 310, the effective elevation length of the transducer element 310 is a first length d₁.

When the processing module operates in the second mode, and both a first set and second set of the transducers contributes to the acoustic pulse emitted by the transducer element 310, the effective elevation length of the transducer element 310 is a second length d₂, which is greater than the first length.

In this way, controlling the number of sets of transducers that contribute to the acoustic pulse emitted by the transducer element thereby controls the effective elevation length of the transducer element.

Figure 4 illustrates a computer-implemented method 400 according to an embodiment of the invention. The method may be implemented an acoustic imaging probe, such as those previously described.

The method 400 comprises a step 410 determining in which mode a processing module is to operate. The determination may be responsive to a user input 490 (e.g. to allow a user to select a mode of the processing module) or some other criteria 495 (e.g. if the processing module is operating according to a particular scheme or strategy, an example of which will be provided later).

In response to determining, in step 410, that the processing module is to operate in the first mode, the method moves to a step 420 of controlling the transducer element (of the acoustic imaging probe) so that only the first set of one or more adjacent transducers emits an acoustic pulse and the transducers of the first set operate synchronously when emitting an acoustic pulse to provide a first combined acoustic pulse.

In response to determining, in step 410, that the processing module is to operate in the second mode, the method moves to a step 430 of controlling the transducer element so that both the first and second sets of one or more adjacent transducers emit acoustic pulses and the transducers of the first and second sets operate synchronously when emitting an acoustic pulse to provide a second combined acoustic pulse.

Steps 420 and 430 represent different modes of the processing module. There may therefore be a method of operating the processing module to be operable in either a first mode (in which step 420 is performed) or a second mode (in which step 430 is performed).

After the transducer element provides a (combined) acoustic pulse, i.e. performs either step 420 or 430, the method may move to a step 440 of receiving, at the transducer element, echoes or reflections of the provided acoustic pulse. The transducer element then generates a receive signal responsive to received echoes or reflections in a step 450. This generated receive signal may be used to generate an acoustic image of the area into which the acoustic pulse was emitted, in a step 460. Methods of generating an acoustic image using a receive signal responsive to echoes of an acoustic pulse will be apparent to the skilled person.

After generating the receive signal, the method may revert back to step 410, as illustrated, and the process for emitting an acoustic pulse may begin again. The skilled person will readily appreciate that the method can be terminated when acoustic imaging is finished (e.g. a user terminates an acoustic imaging process or a memory is full).

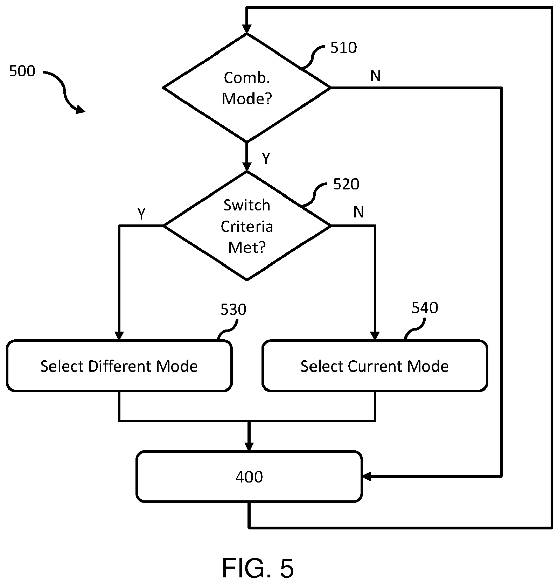

Figure 5 illustrates a method 500 according to a further embodiment of the invention. The method 500 facilitates a combination strategy for the processing module, in which the processing module iteratively switches between operating in the first mode and operating in the second mode.

The method 500 comprises a step 510 of determining whether to operate the processing module according to a combination strategy. This may be performed, for example, by monitoring a user input signal that indicates a desired mode and/or strategy for the processing module.

In response to a negative determination in step 510, i.e. the processing module is not to operate according to the combination strategy, the method simply moves to performing a method 400 (e.g. responsive to a user input selection of the first or second mode).

In response to a positive determination in step 510, the method moves to a step 520 of determining in which mode the processing module is to operate. In particular step 520 may comprise determining whether to switch modes.

In particular step 520 may comprise determining whether some switching criteria has been met. The switching criteria may, for example, comprise a predetermined number of combined acoustic pulses being emitted by the transducers when operating in the current mode, or a predetermined length of time passing since switching to the current mode.

If the switching criteria is met in step 520, the method moves to a step 530 of selecting a different operating mode for the processing mode. Otherwise, the method performs step 540 of selecting the current operating mode for the processing module.

After selecting the operating mode, the processing module performs process 400 (with the selected mode being the mode in which the processing module is to operate).

For a first iteration in which the step 510 determines to operate in the combination strategy, step 520 may comprise arbitrarily selecting a mode in which the processing module to operate, e.g. according to a predetermined selection strategy (e.g. select the first mode).

Step 530 may comprise switching to a different mode according to some predetermined strategy or pattern. Different combination strategies may operate according to different patterns.

For example, a first combination mode may iteratively switch between the first and second modes only. A second, different combination mode (e.g. which may be available if the transducer element comprises three sets of transducers) may switch from the first mode, to the second mode to a third mode, before repeating the pattern. A third, different combination mode (e.g. which may be available if the transducer element comprises three sets of transducers) may switch from the first mode, to the second mode, to a third mode and back to the second mode before repeating the pattern.

The precise pattern may depend upon the number of sets of transducers available, e.g. to control a pattern of the change in size of the elevation length over time.

The switching criteria used in step 520 may be different for different modes of the processing module and/or for different iterations of the method 500.

For example, a combination strategy may iteratively switch between the first and second modes only, where the switching criteria is met for the first mode when a first predetermined number of acoustic pulses have been emitted (since beginning operation in the first mode), and the switching criteria is met for the second mode when a second, different predetermined number of acoustic pulses have been emitted (since beginning operation in the second mode).

As another example, a combination strategy may iteratively switch according to a first predetermined pattern of: first instance of first mode; first instance of second mode; second instance of first mode; and second instance of second mode. The strategy may switch from the first instance of the first mode to the first instance of the second mode in response to a first predetermined number of acoustic pulses being emitted (since the processing module began operating in the first instance of the first mode). The strategy may switch from the first instance of the second mode to the second instance of the first mode in response to a second (preferably different) predetermined number of acoustic pulses being emitted (since the processing module began operating in the first instance of the second mode). The strategy may switch from the second instance of the first mode to the second instance of the second mode in response to a third (preferably different) predetermined number of acoustic pulses being emitted (since the processing module began operating in the second instance of the first mode). The strategy may switch from the second instance of the second mode back to the first instance of the first mode in response to a fourth (preferably different) predetermined number of acoustic pulses being emitted (since the processing module began operating in the second instance of the second mode).

Other suitable examples of combination strategies will be apparent to the skilled person, to facilitate the processing module switching between a different two or more operating modes according to some predetermined pattern.

This method 500 is iteratively repeated, e.g. until some termination criteria is met (e.g. the user terminates an acoustic imaging process or a memory for storing data obtained during the acoustic imaging process is full).

With further reference to Figure 4, the selected mode (in steps 430 or 440) may act as the other criteria 495 for input to the method 400.

The above described embodiments have been described in the context of an a single transducer element. However, it will be appreciated that an acoustic imaging probe may comprise a plurality of transducer elements (e.g. arranged in an array of transducer elements).

Thus, an acoustic imaging probe may comprise a plurality of transducer element, each comprising a first and second set of transducers, wherein the processing module is configured to control the operation of each of the plurality of transducer elements, wherein: when operating in the first mode, the processing module controls each transducer element so that only the first set of one or more adjacent transducers of each transducer element emits an acoustic pulse and the transducers of the first set of each transducer element operate synchronously when emitting an acoustic pulse to provide a first combined acoustic pulse; and when operating in the second mode, the processing module controls each transducer element so that both the first and second sets of one or more adjacent transducers of each transducer element emit acoustic pulses and the transducers of the first and second sets of each transducer element operate synchronously when emitting an acoustic pulse to provide a second combined acoustic pulse.

The plurality of transducer elements may be controlled in a phased array manner, so that each transducer element emits a combined acoustic pulse according a predetermined pattern. Other methods of controlling a plurality of transducer elements (e.g. in a synchronous or a linear manner) will be apparent to the skilled person.

Embodiments of the invention are particularly advantageous when the transducer element (or each transducer element if there are a plurality) is a linear transducer element and/or where the acoustic imaging probe is configured for intravascular ultrasound (IVUS).

Where reference is made to a user input, the acoustic ultrasound probe may comprise a user interface for receiving the user input (e.g. to indicate a desired mode and/or strategy).

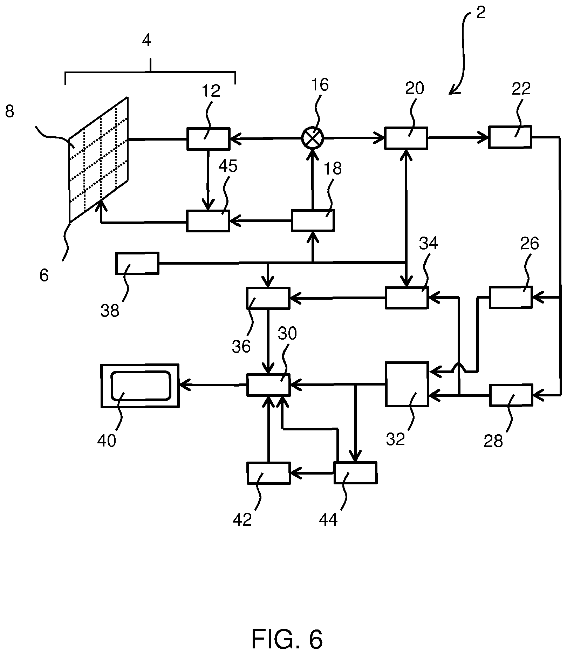

The general operation of an exemplary ultrasound system will now be described, with reference to Figure 6. An ultrasound system is an example of an acoustic imaging system, and the skilled person would be capable of modifying the described ultrasound imaging system to perform non-ultrasound acoustic imaging if desired.

The system comprises an array transducer probe 4 which has an array of transducers 8, or a transducer element 6, for transmitting ultrasound waves and receiving echo information. The transducer element 6 may comprise CMUT transducers; piezoelectric transducers, formed of materials such as PZT or PVDF; or any other suitable transducer technology. In this example, the transducer element array 6 is a two-dimensional array of transducers 8 capable of scanning either a 2D plane or a three dimensional volume of a region of interest. In another example, the transducer element array may be a 1D array, i.e. a linear array.

The transducer element 6, formed of array of transducers 8, is coupled to a microbeamformer 12, which controls reception of signals by the transducer elements. Microbeamformers are capable of at least partial beamforming of the signals received by subarrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer element array is operated directly by the main system beamformer. The transmission of ultrasound beams from the transducer element 6 is directed by a transducer controller 18, an embodiment of the processing module herein described, coupled to the microbeamformer by the T/R switch 16 and a main transmission beamformer (not shown), which can receive input from the user's operation of the user interface or control panel 38. The controller 18 can include transmission circuitry arranged to drive the transducers 8 of the array (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer element array, or a sub-aperture of the transducer element array (e.g. for the present disclosure, depending upon the mode of the transducer controller). The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer. The shifted sub-aperture is then activated and the process repeated until all of the transducer elements of the transducer element array have been activated.

For each line (or sub-aperture), the total received signal, used to form an associated line of the final ultrasound image, will be a sum of the voltage signals measured by the transducers of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subj ect.

One of the functions controlled by the transducer controller 18 is the direction in which beams are steered and focused. Beams may be steered straight ahead from (orthogonal to) the transducer element array, or at different angles for a wider field of view. The steering and focusing of the transmit beam may be controlled as a function of transducer actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducers at the same time, the transducer element array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducers to activate in a series, beginning with the outermost elements and finishing at the central element(s) of the transducer element array, a focal zone would be formed at a given distance away from the probe, in line with the central element(s). The distance of the focal zone from the probe will vary depending on the time delay between each subsequent round of transducer activations. After the beam passes the focal zone, it will begin to diverge, forming the far field imaging region. It should be noted that for focal zones located close to the transducer element array, the ultrasound beam will diverge quickly in the far field leading to beam width artifacts in the final image. Typically, the near field, located between the transducer element array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducers and subject to an electronic time delay before being passed into the system for signal processing. The simplest example of this is referred to as delay-and-sum beamforming. It is possible to dynamically adjust the receive focusing of the transducer element array as a function of time.

Looking now to the function of beam steering, through the correct application of time delays to the transducers it is possible to impart a desired angle on the ultrasound beam as it leaves the transducer element array. For example, by activating a transducer on a first side of the transducer element array followed by the remaining transducers in a sequence ending at the opposite side of the array, the wave front of the beam will be angled toward the second side. The size of the steering angle relative to the normal of the transducer element array is dependent on the size of the time delay between subsequent transducer activations.

Further, it is possible to focus a steered beam, wherein the total time delay applied to each transducer element is a sum of both the focusing and steering time delays. In this case, the transducer element array is referred to as a phased array.

In case of the CMUT transducers, which require a DC bias voltage for their activation, the transducer controller 18 can be coupled to control a DC bias control 45 for the transducer element array. The DC bias control 45 sets DC bias voltage(s) that are applied to the CMUT transducers.

For each transducer element of the transducer element array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducers. In this way, the signals received by thousands of transducers of a transducer element array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and microbubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 6 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer element 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the array transducer element 6 and hence the images produced by the transducer element array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The skilled person would be readily capable of developing a processing system for carrying out any herein described method. Thus, each step of the flow chart may represent a different action performed by a processing system, and may be performed by a respective module of the processing system. The processing system may be or form part of a processing module for an imaging probe.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. An acoustic imaging probe (100, 200 300) comprising:
a transducer element (110, 210, 310) comprising:
a first set (215) of adjacent transducers (211); and
a second set (216) of adjacent transducers, wherein the first set is adjacent to the second set;
wherein each transducer is configured to controllably emit an acoustic pulse and to receive one or more echo signals as response to the emitted acoustic pulse,
a processing module (120, 220, 320) configured to control the operation of the transducer element in at least:
a first mode, in which the processing module is configured to control the transducer element so that only the first set of transducers emits acoustic pulse and the transducers of the first set operate synchronously when emitting acoustic pulses to provide a first combined acoustic pulse; and
a second mode, in which the processing module is configured to control the transducer element so that both the first and second sets of transducers emit acoustic pulses and the transducers of the first and second sets operate synchronously when emitting acoustic pulses to provide a second combined acoustic pulse.

2. The acoustic imaging probe (100, 200, 300) of claim 1, wherein each transducer (211) is configured to require a voltage bias to controllably emit an acoustic pulse, and
the processing module (120, 220, 320) is configured to:
when operating in the first mode, provide a voltage bias to only the first set of transducers; and
when operating in the second mode, provide a voltage bias to the first and second sets of transducers.

3. The acoustic imaging probe of any of claims 1 to 2, wherein the processing module (120, 220, 320) is configured to control the transducer element so that a center frequency of the first combined acoustic pulse is different to a center frequency of the second combined acoustic pulse.

4. The acoustic imaging probe (100, 200, 300) of claim 3, wherein the processing module (120, 220, 320) is configured to control the transducer element so that the center frequency of the first combined acoustic pulse is greater than the center frequency of the second combined acoustic pulse.

5. The acoustic imaging probe of any of claims 1 to 4, wherein the processing module (120, 220, 320) is further configured to be operable according to a combination scheme, in which the processing module iteratively switches between operating in the first mode and operating in the second mode.

6. The acoustic imaging probe of claim 5, wherein, when operating according to the combination scheme, the processing module switches between the first and second mode in response to a predetermined number of combined acoustic pulses being emitted by the transducers.

7. The acoustic imaging probe of any of claims 1 to 6, wherein:
the transducer element further comprises a third set of adjacent transducers, the third set being adjacent to the second set of transducers;
the processing module is further operable in a third mode, in which the processing module is configured to control the transducer element so that the first, second and third sets of transducers emit acoustic pulses and the transducers of the first, second and third sets operate synchronously when emitting acoustic pulses to provide a third combined acoustic pulse.

8. The acoustic imaging probe of any of claims 1 to 7, wherein the processing module is adapted to monitor the first and second sets of transducers to generate a receive signal responsive to the one or more echo signals received by the first and second sets of transducers.

9. The acoustic imaging probe of any of claims 1 to 8, wherein the transducer element comprises capacitive micromachined ultrasonic transducer.

10. The acoustic imaging probe of any of claims 1 to 9, wherein the transducer element comprises piezoelectric transducers.

11. The acoustic imaging probe of any of claims 1 to 10, wherein the processing module is configured to be responsive to a selection of the operating mode of the transducer element by a user input signal.

12. The acoustic imaging probe of any of claims 1 to 11, comprising a plurality of transducer elements, each comprising a first and second set of transducers.

13. A computer-implemented method (400) of controlling an acoustic imaging probe (100, 200, 300) comprising a transducer element (110, 210, 310), the transducer element comprising: a first set (215) of adjacent transducers (211); and a second set (216) of adjacent transducers, the first set adjacent to the second set, wherein each transducer is configured to controllably emit an acoustic pulse and to receive one or more echo signals as response to the emitted acoustic pulse, the computer-implemented method comprising:
controlling the transducer element using at least two modes, including:
a first mode (420), in which the processing module controls the transducer element so that only the first set of transducers emits acoustic pulse and the transducers of the first set operate synchronously when emitting acoustic pulses to provide a first combined acoustic pulse; and
a second mode (430), in which the processing module controls the transducer element so that both the first and second sets of adjacent transducers emit acoustic pulses and the transducers of the first and second sets operate synchronously when emitting acoustic pulses to provide a second combined acoustic pulse.

14. A computer program product comprising computer program code means which, when executed on a computing device having a processing system, cause the processing system to perform all of the steps of the method according to claim 13.

15. A processing module configured to perform the method of claim 13.
